(19) Europäisches Patentamt

European Patent Office

Office européen des brevets

(11) **EP 2 301 513 A2**

(12) **EUROPEAN PATENT APPLICATION**

(43) Date of publication:
**30.03.2011 Bulletin 2011/13**

(51) Int Cl.:
***A61K 6/00*** *(2006.01)*

(21) Application number: **10009845.8**

(22) Date of filing: **17.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **29.09.2009 JP 2009224252**

(71) Applicant: **GC Corporation**
**Itabashi-ku**
**Tokyo 174-8585 (JP)**

(72) Inventors:
• **Kato, Shinichi**
**Itabashi-ku**
**Tokyo 174-8585 (JP)**
• **Sato, Takuya**
**Itabashi-ku**
**Tokyo 174-8585 (JP)**

(74) Representative: **Müller-Boré & Partner**
**Patentanwälte**
**Grafinger Straße 2**
**81671 München (DE)**

(54) **Tooth coating composition**

(57) To provide a tooth coating composition capable of maintaining dental caries preventing effects of CPP-ACP, such as recalcification effect and demineralization-inhibiting effect, for a long period of time, a tooth coating composition is made not to contain water, but to include (a) 0.5 to 20% by weight of a casein phosphopeptide-amorphous calcium phosphate complex (CPP-ACP) and/or a casein phosphopeptide-amorphous calcium fluoride phosphate complex (CPP-ACFP), (b) 5 to 70% by weight of a coating film- forming polymer, (c) 5 to 70% by weight of an organic solvent selected so as to contain alcohol, (d) 0.01 to 10% by weight of a fluorine compound, and (e) 0.1 to 20% by weight of a thickener, the tooth coating composition being used by coating on a tooth.

**Description**

The present invention relates to a tooth coating composition capable of coating on a tooth so as to form a film and keeping a dental caries preventing effect for a long period of time.

A casein phosphopeptide-amorphous calcium phosphate complex (it will be simply called CPP-ACP below) is a material consisting of a complex of peptide originating in protein (casein) of a cow's milk and an inorganic material (amorphous calcium phosphate). Because of including calcium and phosphorus as inorganic components of a tooth in its constituent, the CPP-ACP has an effect to promote recalcification of a tooth.

In prevention of a dental caries by the CPP-ACP, there are two processes which are a recalcification action to a dentin and demineralization-inhibiting action to a dentin. The recalcification action to a dentin is generated by continuously supplying ions of calcium and phosphorus into a demineralization lesion under an enamel surface layer by the CPP-ACP. The demineralization-inhibiting action to a dentin is generated in a condition that solubility of calcium phosphate of a dentin enamel surface layer is greatly decreased and a tooth becomes to be hardly dissolved, as a result of ion concentrations of calcium and phosphorous being kept in a supersaturated state in saliva in an oral cavity by ions of calcium and phosphorous originated in the CPP-ACP. A casein phosphopeptide-amorphous calcium fluoride phosphate complex (it will be simply called CPP-ACFP below), which includes fluoride, has also effect for preventing dental caries.

For example, Japanese Translation of PCT Publication No. 2002-500626 and Japanese Translation of PCT Publication No. 2002-540129 disclose products for auxiliary preventing a dental caries such as foods, for example, a gum and a candy, and oral cavity caring compositions, for example, a dentifrice and a mouth wash, in which these products are made to include the CPP-ACP and the CPP-ACFP. However, in order to fully exercise the dental caries preventing effect of the CPP-ACP in an oral cavity, it is necessary to make the CPP-ACP stay for a long time. For example, in a case of the gum including the CPP-ACP, it is necessary as a standard to take two pieces (3g) simultaneously, 4 times a day, for approximately 20 minutes, and further to continue it for 2 weeks.

Since it is hard in a general life style to take these products many times a day, for example, Japanese Patent Application Laid-Open No. 2005-145952 discloses a pasty or creamy dental caries preventing composition including CPP-ACP. This composition can exercise the dental caries preventing effect of the CPP-ACP even by taking the composition once a day.

However, since this dental caries preventing composition is a hydrophilic pastey composition, the composition is dissolved with saliva etcetera in an oral cavity. Thus, there are problems that the composition flows away in several minutes when directly coated on a tooth, and at most in several hours even when a tray is used. Therefore, it is required to produce a composition capable of being held on a tooth for a long period of time when being coated on a tooth once and continuously exercising dental caries preventing effects such as recalcification and demineralization-inhibiting of CPP-ACP.

The present invention is directed to a tooth coating composition capable of keeping dental caries preventing effects of CPP-ACP, such as a recalcification effect and a demineralization-inhibiting effect, for a longer period of time.

According to an aspect of the present invention, a tooth coating composition of the present invention does not include water, but includes (a) 0.5 to 20% by weight of a casein phosphopeptide-amorphous calcium phosphate complex (CPP-ACP) and/or a casein phosphopeptide-amorphous calcium fluoride phosphate complex (CPP-ACFP), (b) 5 to 70% by weight of a coating film- forming polymer, (c) 5 to 70% by weight of an organic solvent selected to contain alcohol, (d) 0.01 to 10% by weight of a fluorine compound, and (e) 0.1 to 20% by weight of a thickener. The tooth coating composition is used by coating on a tooth.

The tooth coating composition according to the present invention is an excellent tooth coating composition which exercises the demineralization-inhibiting effect of CPP-ACP and/or CCP-ACFP for a long period of time when coated on a tooth, and can continuously promote the dental caries preventing effect such as recalcification effect.

A tooth coating composition according to the present invention does not include water as a constituent component. When the composition includes water, a film formed after coating of the composition on a tooth surface is weak. Thus, there are problems that the film is broken easily, the tooth coating composition leaves out, and so forth.

A casein phosphopeptide-amorphous calcium phosphate complex (CPP-ACP) and/or a casein phosphopeptide-amorphous calcium fluoride phosphate complex (CPP-ACFP) are not limited especially when these are permitted as a food or for a dental purpose. For example, Japanese Translation of PCT Publication No. 2002-500626 discloses a method for preparing the CPP-ACP and/or (CPP-ACFP). In this method, phosphopeptide is mixed with calcium, inorganic phosphoric acid, and arbitrarily fluorine, and the mixture is filtrated and dried. The composition prepared by this method is preferably used from a view point of having proper composition and degree of crystallinity.

A blending amount of the casein phosphopeptide-amorphous calcium phosphate complex (CPP-ACP) and/or the casein phosphopeptide-amorphous calcium fluoride phosphate complex (CPP-ACFP) is 0.5 to 20% by weight with respect to the whole tooth coating composition, and is preferably 1 to 12% by weight. If the amount is less than 0.5% by weight, calcium and phosphorous are supplied insufficiently, and thus a dental caries preventing effect cannot be obtained. If the amount exceeds 20% by weight, viscosity of the tooth coating composition becomes too high, and preservability deteriorates.

The coating film- forming polymer is a necessary component for forming a stable film including the CPP-ACP and/or the CPP-ACFP on a tooth after coating of the tooth coating composition on the tooth, keeping the film on the tooth, and continuously exercising various dental caries preventing effects. As for the coating film-forming polymer (b), natural materials such as shellac, rosin, pullulan, zein, carnauba wax, beeswax, hemilose and gelatin or a derivative thereof, and organic synthetic materials such as polyethylene glycol, polypropylene glycol, polyethylene oxide, polyvinyl alcohol, polyvinyl acetate, polyvinyl chloride, a vinyl chloride-vinyl acetate copolymer, polyacrylic acid, polymaleic acid, an alkyl acrylate polymer, an alkyl methacrylate polymer, a methoxymethylene / maleic anhydride copolymer, a polyethylene glycol / methylenediisocyanatocyclohexane copolymer and a polypropylene glycol / methylenediisocyanatocyclohexane copolymer, for example, can be used. Of course, two or more kinds can be combined to be used.

A blending amount of the coating film- forming polymer is 5 to 70% by weight with respect to the whole composition and is preferably 10 to 60% by weight. If the amount is less than 5% by weight, the film formed after coating is insufficient, and thus there occur problems that the film leaves out easily and so forth. If the amount exceeds 70% by weight, preservation stability of the tooth coating composition is decreased.

After coating of the tooth coating composition of the present invention on the tooth, the tooth coating composition is dried, naturally dried or dried with compressed air so that a coating film is formed on the tooth surface. In order to properly carrying out coating, drying, and film formation, it is necessary for the tooth coating composition of the present invention to include an organic solvent (c) selected so as to contain alcohol as a volatile component.

Alcohol as the organic solvent is indispensable. As for the alcohol, ethanol, methanol, propanol, isopropanol, butanol, isobutanol, pentanol, isopentanol, oleyl alcohol, octanol, benzyl alcohol, methyl cellosolve, ethyl cellosolve, and the like can be used. Further, two or more kinds of selected alcohols can be combined to be used.

A blending amount of the alcohol is 30 to 95% by weight with respect to the whole organic solvent, and is preferably 45 to 70% by weight. When the amount is less than 30% by weight, sufficient volatile effects for film forming cannot be obtained. When the amount exceeds 95% by weight, viscosity in the composition tends to be too low.

The organic solvent used in the present invention other than the alcohol could be one kind or a combination of two or more kinds selected from acetone, methyl ethyl ketone, methyl isobutyl ketone, styrene, n-pentane, isopentane, tetramethyl methane, n-hexane, cyclohexane, n-heptane, 2-methylhexane, 3-methylhexane, dimethylpentane, trimethylbutane, butyl glycol, methyl diglycol, ethyl diglycol, butyl diglycol, dimethyl diglycol, 1-methoxy-2-propanol, methyl dipropylene glycol, 3-methoxybutanol, tetrahydrofuran, ethyl acetate, propyl acetate, isopropyl acetate, butyl acetate, isobutyl acetate, butyl cellosolve acetate, ethyl carbitol acetate, and the like.

A blending amount of the organic solvent including alcohol is 5 to 70% by weight with respect to the whole composition, and is preferably 10 to 50% by weight. When the amount is less than 5% by weight, the coating film-forming polymer is hard to be dissolved with the organic solvent, so that a coating operation comes to be hard. When the amount exceeds 70% by weight, preservation stability of the composition decreases.

The tooth coating composition according to the present invention includes a fluorine compound (d) for giving a more preferable recalcification effect. A blending amount of the fluorine compound (d) is preferably 0.01 to 10% by weight, and more preferably 0.1 to 6% by weight. If the amount is less than 0.01% by weight, the sufficient recalcification effect is obtained hardly. If the amount exceeds 10% by weight, there is a problem in safety to a human body. As for the fluorine compound, one kind or a combination of two or more kinds selected from sodium fluoride, tin fluoride, and monofluoro sodium phosphate can be used.

It is necessary for the tooth coating composition according to the present invention to include a proper thickener for stabilizing the blended materials in the composition. The thickener (e) could be one kind or a combination of two or more kinds selected from aerogel, methyl cellulose, carboxymethylcellulose, carboxymethylcellulose sodium, carboxymethylcellulose calcium, carboxymethylcellulose pottasium, hydroxyethyl cellulose, hydroxyethyl cellulose, hydroxypropyl cellulose, polyvinyl pyrrolidone, acacia gum, Arabian gum, guar gum, carob bean gum, tara gum, tamarind seed gum, tragacanth gum, karaya gum, carrageenan, chitin, chitosan, chitosamine, xanthan gum, gellan gum, curdlan, carbopol, Lucentite, sodium alginate, propylene glycol alginate, sodium starch glycolate, sodium starch phosphate, and sodium polyacrylate, and the like.

A blending amount of the thickener with respect to the whole composition is 0.1 to 20% by weight, and is preferably 0.5 to 10% by weight. When the amount is less than 0.1% by weight, preservation stability of the composition decreases. When the amount exceeds 20% by weight, viscosity of the composition comes to be too high, so that the composition is hardly coated on a tooth surface.

The tooth coating composition according to the present invention can include an antimicrobial agent in order to give a sterilization effect to a tooth face by coating of the composition to the tooth. The antimicrobial agent (f) could be one kind or a combination of two or more kinds selected from sodium azulenesulfonate, ε-aminocaproic acid, allantoin, allantoin chlorohydroxyaluminum, allantoin dihydroxyaluminum, epidihydrocholesterol, dihydroxycholesterol, glycyrrhizinic acid, diammonium glycyrrhizinate, disodium glycyrrhizinate, trisodium glycyrrhizinate, dipotassium glycyrrhizinate, monoammonium glycyrrhizinate, chlorhexidine gluconate, β-glycyrrhetinic acid, isopropyl methyl phenol, cetylpyridinium chloride, dequalinium chloride, benzalkonium chloride, a benzalkonium chloride liquid, benzethonium chloride, a benzethonium chloride liquid, an alkyldiaminoethylglycine hydrochloride liquid, chlorhexidine hydrochloride, chlorhexidine gluconate, triclosan, pyridoxine hydrochloride, acetic acid d1-α-tocopherol, nicotinic acid d1-α-tocopherol, zeolite, methyl paraoxy-benzoate, ethyl paraoxy-benzoate, propyl paraoxy-benzoate, butyl paraoxy-benzoate, benzyl paraoxy-benzoate, disodium dihydrogen pyrophosphate, sodium pyrophosphate, anhydrous sodium pyrophosphate, sodium hydrogen phosphate, trisodium phosphate, sodium polyphosphate, stearyl dimethyl benzyl ammonium chloride, lauryl dimethyl benzyl ammonium chloride, distearyl dimethyl ammonium chloride, stearyl trimethyl ammonium chloride, cetyl trimethyl ammonium chloride, lauryl trimethyl ammonium chloride, stearyl trimethyl ammonium bromide, cetyl trimethyl ammonium bromide, benzalkonium chloride, benzethonium chloride, dequalinium chloride, chlorhexidine chloride, chlorhexidine gluconate, chlorhexidine acetate, hexetidine, alkyldiaminoethylglycine hydrochloride, domiphen bromide, lauroylcolaminoformylmethylpyridinium chloride, cetyltrimethylammonium saccharin, triclosan, 2,2'-dihydroxy-5,5'-dibromo-diphenyl ether, 4',5-dibromosalicylanilide, 3,4',5-trichlorosalicylamide, 3, 4' , 4-trichlorocarbanilide, 3-trifluoromethyl-4, 4-dichlorocarbanilide, 2-isopropyl-5-methyl-phenol (thymol), 2-methoxy-4- (2-propenyl) -phenol (eugenol), methyl-p-chlorophenol, ethyl-p-chlorophenol, hexylresorcinol, methyl-resorcinol, methyl-resorcinol, dodecyldiaminoethylglycine, benzoate, lauroyl sarcosine, lauryl sulfate, and the like.

A blending amount of the antimicrobial agent (f) is preferably within a range from 0.001 to 3% by weight, and more preferably 0.01 to 0.5% by weight. If the amount is less than 0.001% by weight, the sufficient antimicrobial effect is hardly obtained. If the amount is more than 3% by weight, there is a problem in safety to a human body.

As for a state of the tooth coating composition according to the present invention, any one of a liquid state, a gel state, a paste state, and a cream state can be used. As for a method for coating the composition on a tooth, an applying method using a brush or a swab, or a spraying method using a sprayer can be considered and any of these methods can be used.

In addition, the tooth coating composition according to the present invention can properly include additives such as a coloring agent, a flavoring agent and a bleaching agent.

[Example]

The present invention will be described in detail below with examples and comparative examples, but the present invention is not limited to these examples.

Blending ratios of compositions of Examples 1 to 7 and Comparative examples 1 to 3 are shown in Table 1 collectively. Units of values in Table 1 are parts by weight.

<Evaluation of tooth face persistence -1, Long-term remaining test>

(1) A cow' s tooth was embedded with a dental resin, and a cow's tooth enamel was mirror polished by a water-resistant polishing paper.

(2) A tooth face coating composition blended with the compositions shown in Table 1 was coated on the mirror polished cow's tooth enamel with a brush so as to cover the whole tooth face, and dried with compressed air at a low pressure so as to form a film on the tooth face.

(3) The composition was held for 1 hour at a temperature of 37°C and a humidity of 100%, and dipped in distilled water at 37°C for 3 weeks. Then, a remaining degree of the film on the tooth face was confirmed.

◎: Completely remained ~ mostly remained
○: 50% or more remained
△: Less than 50% remained partially
×: Mostly peeled or dissolved

< Evaluation of tooth face persistence -2, Abrasion test>

(1) A cow's tooth was embedded with a dental resin and a cow's tooth enamel was mirror polished by a water-resistant polishing paper.

(2) A tooth face coating composition blended with compositions shown in Table 1 was coated on the mirror polished cow's tooth enamel with a brush so as to cover the whole tooth face, and dried with pressurized air at low pressure so as to from a film on the tooth face.

(3) The composition was held for 1 hour at a temperature of 37˚C and a humidity of 100%, and subjected to a 5000 time abrasion test with a brush abrasion testing machine. Then, a remaining degree of the film on the tooth face was confirmed.

◎: Completely remained ~ mostly remained
○: 60% or more remained
△: Less than 60% remained partially
×: Mostly peeled

<Evaluation of a recalcification effect>

(1) A cow's tooth was cut to have a thickness of 1mm and the cow's tooth enamel side was polished by a water-resistant polishing paper of #1500. The cut tooth was masked except a part of about 2mm on the enamel side of the cut tooth so as to make a testing body.

(2) The testing body was dipped in a demineralization gel (lactic acid: 0.1M, calcium chloride: 1.5mM, dipotassium phosphate: 0.9mM, CMC-Na: 1% by weight, pH: 4.8) for 4 days so as to generate an artificial dental caries.

(3) The demineralized testing body was observed by a transmission method using a X-ray CT device so as to measure the amount of mineral loss.

(4) A tooth coating composition blended with compositions shown in Table 1 was coated on the artificial dental caries portion of the testing body, and dried so as to form a film.

(5) Each testing body was dipped for 2 weeks in artificial saliva (the artificial saliva was changed everyday). Then, the testing body was observed again by a transmission method using a X-ray CT device so as to measure the amount of mineral loss after recalcification.

(6) A recalcification rate was calculated by the following formula.

$$\text{Recalcification rate (\%)} = \{(\text{the amount of mineral loss at starting time} - \text{the amount of mineral loss after recalcification}) / \text{the amount of mineral loss at starting time}\} \times 100$$

(Larger values indicate more recalcification.)

These results were shown in Table 1 collectively.

<Evaluation of preservation stability>

The tooth coating composition blended with compositions shown in Table 1 was stored for 3 days at a temperature of 60˚C, and then observed. The composition having no liquid separation, no precipitation, and no deposition was indicated with "Good". The results were shown in Table 1 collectively.

Table 1

| | Example 1 | Example 2 | Example 3 | Example 4 | Example 5 | Example 6 | Example 7 | Comparative example 1 | Comparative example 2 | Comparative example 3 |
|---|---|---|---|---|---|---|---|---|---|---|
| (a) CPP-ACP and/or CPP-ACFP | | | | | | | | | | |
| CPP-ACP | 5 | | 2 | 5 | 3 | 4 | 2 | | 3 | |
| CPP-ACFP | | 5 | 2 | | 4 | 4 | 4 | 2 | | 2 |
| (b) Coating film-forming polymer | | | | | | | | | | |
| Shellac | 30 | | | | | | 15 | | | |
| Zein | | 25 | 12 | | | | | | | |
| Rosin | | | 20 | | | 10 | | | 40 | |
| Polyvinylacetate | | | | 45 | | 34 | 15 | 47 | | |
| Alkyl methacrylate polymer | | | | | 35 | | 15 | | | 32 |
| (c) Organic solvent selected so as to contain alcohol | | | | | | | | | | |
| Alcohol | | | | | | | | | | |
| Ethanol | 50 | 40 | | 30 | | | 15 | | | 20 |
| Isopropanol | | | 40 | | | | 15 | | | |
| Oleyl alcohol | | | | | 43 | 30 | | | | |
| Organic solvent other than alcohol | | | | | | | | | | |
| Isopentane | | 10 | | | | | | | | |
| n-hexane | | | 20 | | 10 | | | 46 | | |
| Ethyl diglycol | | | | 20 | | | | | | |
| Tetrahydrofuran | | | | | 15 | | 15 | 10 | 52 | |

(continued)

| | Example 1 | Example 2 | Example 3 | Example 4 | Example 5 | Example 6 | Example 7 | Comparative example 1 | Comparative example 2 | Comparative example 3 |
|---|---|---|---|---|---|---|---|---|---|---|
| (d) Fluorine compound | | | | | | | | | | |
| Sodium fluoride | 1 | | | 3 | | | 2 | | | |
| Monofluoro sodium phosphate | | 6 | | | 2 | | | 3 | | 3 |
| Tin fluoride | | 2 | | | | 3 | | | 3 | |
| (e)Thickener | | | | | | | | | | |
| Aerosil R972 | | | | | | | | | | 3 |
| Aerosil A200 | 4 | | | | | | | | | |
| Hydroxypropyl cellulose | | | 2 | | | | | 1 | | 5 |
| Polyvinyl pyrrolidone | | 4 | | | | 1 | 2 | 2 | | |
| Sodium alginate | | | | 2 | | | | | | |
| (f) Antimicrobial agent | | | | | | | | | | |
| Chlorhexidine hydrochloride | | | | | | 0.01 | 0.08 | | | |
| Cetylpyridinium chloride | | | | | | | 0.02 | | | |
| Other additives | | | | | | | | | | |
| Titanium oxide | | | | | | 1 | 2 | | | |
| Water | | | | | | | | | | 40 |
| Xylitol | | | | | | | 1 | | | |
| Saccharin sodium | | | | | | 1 | 0.4 | | | |
| Lemon flavor | | | | | | | 0.5 | | | |
| Total | 100 | 100 | 100 | 100 | 100 | 100 | 100 | 100 | 100 | 100 |

(continued)

| | Example 1 | Example 2 | Example 3 | Example 4 | Example 5 | Example 6 | Example 7 | Comparative example 1 | Comparative example 2 | Comparative example 3 |
|---|---|---|---|---|---|---|---|---|---|---|
| Long-term remaining test [persistence after 3 weeks] | ◎ | ◎ | ◎ | ◎ | ◎ | ◎ | ◎ | △ | △ | × |
| Abrasion test [persistence after 5000 time testing] | ◎ | ◎ | ◎ | ◎ | ◎ | ◎ | ◎ | × | × | × |
| Recalcification rate (%) | 43.3 | 49.8 | 52.3 | 38.8 | 43.9 | 58.7 | 56.9 | 15.6 | 18.4 | 36.1 |
| Preservation stability (stored at 60°C for 3 days) | Good | Good | Good | Good | Good | Good | Good | Precipitation | Precipitation | Browning |

Clearly from Table 1, the tooth coating composition of the present invention could form a coating film having long-term stability and high abrasion resistance by coating of the composition on the tooth and drying. Further, the recalcification effect was exercised by actions of CPP-ACP, CPP-ACFP, and a fluorine compound. Therefore, it was confirmed that the tooth coating composition had an excellent property.

**Claims**

1. A tooth coating composition not containing water, wherein the composition comprises:

   (a) 0.5 to 20% by weight of a casein phosphopeptide-amorphous calcium phosphate complex (CPP-ACP) and/or a casein phosphopeptide-amorphous calcium fluoride phosphate complex (CPP-ACFP);
   (b) 5 to 70% by weight of a coating film- forming polymer;
   (c) 5 to 70% by weight of an organic solvent selected so as to contain alcohol;
   (d) 0.01 to 10% by weight of a fluorine compound; and
   (e) 0.1 to 20% by weight of a thickener,

   the tooth coating composition being used by coating on a tooth.

2. The tooth coating composition as claimed in claim 1, wherein the composition further comprises (f) 0.001 to 3% by weight of an antimicrobial agent.

3. The tooth coating composition as claimed in-claim 1 or 2, wherein a coating film- forming polymer is one kind or a combination of two or more kinds selected from shellac, rosin, pullulan, zein, carnauba wax, beeswax, hemilose, gelatin or a derivative thereof, polyethylene glycol, polypropylene glycol, polyethylene oxide, polyvinyl alcohol, polyvinyl acetate, polyvinyl chloride, a vinyl chloride-vinyl acetate copolymer, polyacrylic acid, polymaleic acid, an alkyl acrylate polymer, an alkyl methacrylate polymer, a methoxymethylene / maleic anhydride copolymer, a polyethylene glycol / methylenediisocyanatocyclohexane copolymer, and a polypropylene glycol / methylenediisocy-anatocyclohexane copolymer.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002500626 W
- JP 2002540129 W

- JP 2005145952 A